# EUROPEAN PATENT SPECIFICATION

(11) **EP 1 661 524 B1**
(45) Date of publication and mention of the grant of the patent: **02.01.2008**
(21) Application number: 05425779.5
(22) Date of filing: 08.11.2005
(51) Int. Cl.: A61B 17/64, F16B 7/04

(54) **Multifunctional fixation device for reduction and stabilisation of fractures, particularly to be used with Kirschner wire technique**
Multifunktionale Fixationsvorrichtung zur Repositionierung und Stabilisierung von Knochenfrakturen insbesondere mit Kirschnerdrähten
Appareil multifonctionnel pour la réduction et l'immobilisation de fractures utilisable, en particulier, avec des broches de Kirschner

(30) Priority: 24.11.2004 IT RM20040575
(43) Date of publication of application: 31.05.2006
(73) Proprietor: Tangari, Mario, 00142 Roma (IT)
(72) Inventor: Tangari, Mario, 00142 Roma (IT)
(74) Representative: Iannone, Carlo Luigi

(56) References cited:
- EP-A- 0 350 925
- DE-C1- 4 103 494
- US-A- 5 624 440

## Description

The present invention relates to a multifunctional fixation device for reduction and stabilisation of fractures, particularly to be used with Kirschner wire technique.

More specifically, the invention relates to a (external, internal and hybrid) multifunctional fixation device suitable for stabilisation after reduction of fractures by the Kirschner wire technique, thus permitting the use of this technique also for multiple fractures, diaphyseal fractures, epiphyseal fractures, simple fractures and comminute fractures.

As it is well known, presently Kirschner wire technique is widely used for reduction and stabilisation of fractures. This technique provides insertion of metallic wires or fiches within the bone tissue, by a radioscopy control, after the recomposition of the fracture. Said wires or fiches are comprised of steel wires having one threaded end, used to obtain a greater fixing stability, particularly in case of osteoporosis osteosynthesis.

Said wires generally provide the end introduced within the bone tissue having a "sharpened" shape or, as in the present invention, a "mouthpiece of a flute" shape, in order to facilitate penetration of diaphyseal channel and to avoid wrong perforation direction.

Introduction of said wires or fiches within the bone tissue occurs by a light rotatory action, by employing suitable spindles or drilling machines. Then, suitable fixation devices outside block parts of wires or fiches projecting from patient epidermis.

The above mentioned wires can have a different diameter to be suitable to the bone segment to be subjected to treatment, such as metacarpal, metatarsus, humerus, radio, phalanx, cubitus, shinbone, phemour, ecc.

At present, different kinds of outer fixation devices exist on the market, exclusively realised for the inner synthesis of meta-epiphyseal fractures (bone ends). Among the latter devices, a first kind of device is realised as a pair of plate shaped elements, providing one or more fixing dowels. These elements provide two or more parallel channels. The surgeon, after having introduced the wires within the bone tissue, places two wires on a pair of channels of one of said plate shaped elements, and then superimposes the second element. Finally, he fixes said two elements by screws.

This solution, to be applied, requires that the wires are substantially parallel each other. Therefore, said technique is only useful for epiphyseal fractures. In case it is necessary treating a multiple fracture, said fixation devices do not permit bridge quick couplings. In other words, they are particularly inconvenient for application with diaphyseal fractures and small segment fractures such as finger ungual phalanx. Particularly, they can only be used for the so-called inner synthesis, i.e. in case wires are introduced within the diaphyseal channel. They cannot be easily applied, but in particular cases and very difficulty, for outer synthesis, i.e. those requiring the introduction of wires and fiches outside the diaphyseal channel ad that would require bridge connections or when a plurality of wires is used, placed on different planes or segments.

It should be noted that the above-mentioned solution obliges the surgeon to fold the wires in order to make them parallel each other by suitable forceps. In case of particularly delicate fractures, or for very small bones, it is very cumbersome keeping the fractured bone blocked while wires or fiches are bent.

Furthermore, wire blocking system, that must be beforehand laid down on the element, is rather inconvenient, slow and not versatile, also taking into consideration that many fracture reductions occurs with the Hospital First Aid, thus under urgency conditions.

Not being able to make bridge fixing does not permit positively solving the problem of having the bone sliding along wires or fiches that are also present for epiphyseal fracture. This problem generally involves that the surgeon makes many checks of the fracture in order to avoid that it breaks out.

An alternative, but substantially similar, solution, since it is suitable for the meta-epiphyseal fractures, and possibly also with retrograde mode of some of them (shoulder) is due to the presence of pyramid shaped clamps. This solution permits fixing of each one of the lateral surfaces of said pyramid with only one wire within a suitable channel. Each surface of said pyramid further provides a wire blocking screw.

This solution does not completely solve the problem of handling wires by the surgeon in order to lay them down parallelly along the channels of the pyramid surface and of the inconvenient positioning for the patient.

It is well evident that the above procedures require much time to the surgeon and do not ensure an optimum result in any situation and kind of fracture.

En example of a fixing device for osteosynthesis is known from patent application DE 41 03 494 C1 (The preamble of claim 1 is based on this document), which discloses an external fixing device for the mutually fixing bones or bone fragments by means of bone screws insertable into the bones, said bone screws having a stem extending outwardly of the bones, and a retainer frame, on which retaining devices are provided for rigidly retaining the stems of said bone screws, characterised in that the stems are releasably connected with the point portions of the bone screws inserted into the bones.

EP 350 925 A1, which again forms part of the state of the art, describes an external clamping device for a bone fracture for mechanical clamping of the fracture parts of a fractured bone. Said clamping device has bone-supporting bars, which are inserted transversely into the fracture parts and can be clamped outside the bone between two clamping jaws which are provided with grooves and which together form the first pivot member of a hinge, whose second pivot member is provided with connection rods for mechanical connection of several hinges. The second pivot member consists of two clamping jaws provided with grooves complementary to the connection rods.

A disadvantage with the devices known from the state of the art, is that they do not allow a surgeon to couple the wires to the fixation device reliably, removably and slidingly, so that he can determine the preferred position of the device after the coupling of the wires, before a final tightening.

Furthermore, another drawback of the known devices is that they can couple few wires. Hence, they cannot be used in complex external fixation structures.

The solution according to the present invention provides a fixation device, which allows a lateral and provisional coupling, allowing a surgeon to realize complex structure without efforts.

In view of the above, it is well evident the needing of having available a fixation device resolving the above mentioned drawbacks and that is suitable for any situation.

Therefore, it is an object of the present invention that of permitting carrying out inner, outer and hybrid synthesis, by a quick closure mechanism, thus ensuring a high versatility.

Further object of the present invention is that of providing fittings to be associated to the outer fixation device, thus increasing the use possibilities.

It is therefore specific object of the present invention a multifunctional fixation device for reduction and stabilisation of fractures, particularly to be used with Kirschner wire technique, said technique providing at least two wires introduced within the bone tissue. The device according to the invention is disclosed in claim 1. Further embodiments are in the dependant claims.

The present invention will be now described, for illustrative but not limitative purposes, according to its preferred embodiments, with particular reference to the figures of the enclosed drawings, wherein:
figure 1 shows an exploded lateral view of an outer fixation device for reduction of fractures according to the invention;
figure 2 shows a lateral view of the outer fixation device according to figure 1 fixing a Kirschner wire;
figure 3 shows a perspective view of the outer fixation device according to figure 1 fixing two Kirschner wires;
figure 4 is a top view of a first upper element of the outer fixation device according to figure 1;
figure 5 shows a top view of a blocking element of the outer fixation device according to figure 1;
figure 6 shows a bottom view of a second element of the outer fixation device according to figure 1;
figure 7 shows a pair of tools for application of the outer fixation device according to the invention;
figure 8 shows an exploded lateral view of a second embodiment of an outer fixation device according to the invention comprising two blocking elements;
figure 9 shows a lateral view of the outer fixation device according to figure 8;
figure 10 shows the application of outer fixation devices according to the present invention during ostheosynthesis of metacarpal and of cubitus;
figure 11a shows a lateral view of a first embodiment of a clamp for fixing wires or fiches; and
figure 11b shows a lateral view of a second embodiment of a clamp for fixing wires or fiches.

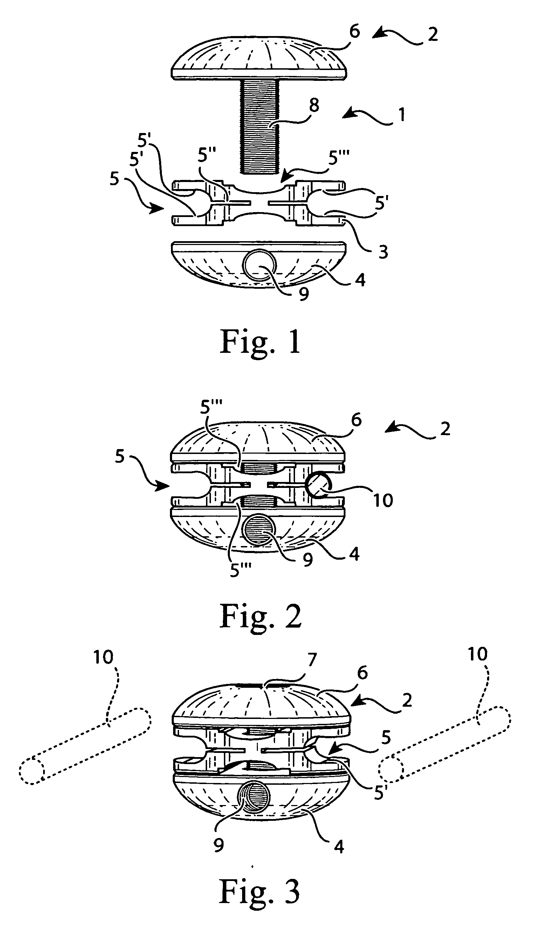

Making reference to figure 1, it is possible observing an embodiment of the outer fixation device 1 according to the present invention. Said device 1 provides a screw 2, a blocking element 3 and a nut element 4. Said blocking element 3 is placed between said screw 2 and said nut element 4, and provides parallel lateral seats 5 for wires fiches, so that when screw 2 is tightened on the nut 4, seats 5 tighten fixing wires or fiches.

Observing in greater detail figures 1, 2 and 3, it can be noted that said screw 2, realizes a soket head screw, has a dome shaped head 6, at the top of which a soket 7 for a socket head screw is provided. The thread of the stem 8 of said screw 2 can have different length and pitch, according to the application needs.

Said nut element 4 has a threaded lateral hole 9, within which a tool having a threaded end is introduced, suitable to handle said nut 4 during the osteosynthesis operation and to prevent anomalous rotation during tightening.

Said blocking element 3 has lateral seats 5, with suitable sizes for fixing different wires or fiches 10. Each of said seats 5 is formed by two upper and lower grooves 5' that, after having introduced a wire 10, make the wire "snap" so as to make the introduction in operative position safe.

Wires 10 are introduced laterally, as it can be observed in greater detail in figure 3. This solution allows pre-assembling the outer fixation device before introduction of wires or fiches 10. In this way, by keeping the nut element blocked, and consequently blocked the whole outer fixation device, by a tool through the threaded hole 9, it is possible to easily introduce wires or fiches 10 within seats 5, as a consequence of the snapping caused by grooves 5', thus ensuring the proper introduction of wires or fiches 10, then, a socket head screw 2 is thightened by a suitable tool.

Seats 5 also have a slit 5", amenable to close when tightening the screw 2 with the nut element 4, and upper and lower channels 5"', promoting the closure of seats 5 of said blocking element 3 on said wires or fiches 10.

After screwing said screw 2 on said nut element 4, it is possible to introduce a further screw (not shown) within the threaded hole 9, that can also be used to block the screw 2 by perpendicular contact on threaded stem 8, so as to prevent loosening of fixation device 1.

In a further embodiment it is possible to provide a through channel (not shown in figures 1, 2, 3) parallel with respect to the diameter of said nut element 4, perpendicular with respect to said hole 9. Said channel can be used for introduction of a wire 10, under particular surgical conditions (as in case of a patella fracture or for tendon lesions). Said further wire 10 could be fixed by a screw (not shown) screwed within the hole 9.

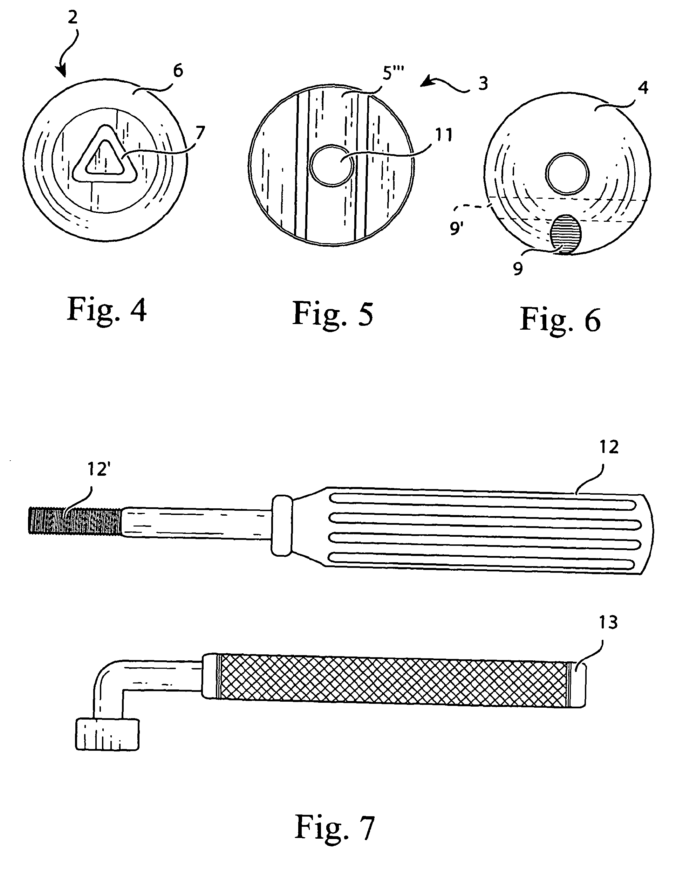

Figure 4 shows the upper soket 7 for introduction of a socket head screw key. In the embodiment shown, said soket 7 has a triangular perimeter.

Figure 5 shows a top view of the blocking element 3, wherein channels 5"' are easily individuated and hole 11 for passage of the threaded stem 8 of said screw 2.

Figure 6 shows a bottom view of the nut element 4, wherein it is possible to particularly individuate the hole 9 and the possible channel 9' (shown by a hatched line).

Figure 7 shows a tool 12 having a threaded end 12', to be introduced within hole 9 of the nut element 2. Furthermore, this figure shows a socket head screw key 13 to rotate the head 6 of the screw 2 by the soket 7.

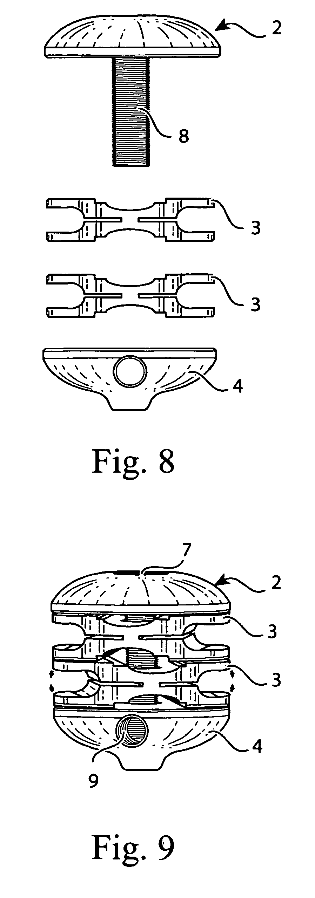

Figure 8 shows a further embodiment of the outer fixation device according to the present invention. Particularly, a screw 2, a nut element 4 and a pair of blocking elements 3, one above the other, it can be observed.

In the embodiment shown, nut element 4 can receive a longer threaded stem 8.

Figure 9 shows the outer fixation device of figure 8 assembled. Particularly, it is possible to observe that two blocking elements 3 can be rotated with respect to each other according to every angle. Thus it is possible to block pairs of wires or fiches 10 with a divergent angle. This permits making very versatile the outer fixation device 1, thus allowing reducing simple or comminute epiphyseal fractures, simple or comminute diaphyseal fractures, even by realisation of bridge connections.

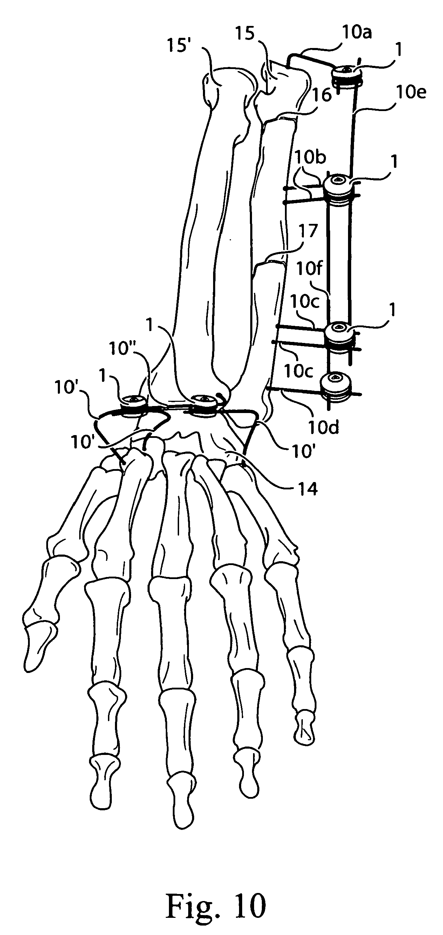

Making reference to figure 10, it is possible to observe the use of a plurality of said outer fixation devices 1 for reducing a carpus 14 fracture and a cubitus 15 multiple fracture.

As far as the carpus 14 fracture is concerned, it is possible to apply three L shaped fiches 10', two of them having one end fixed to the carpus 14 bones and the other one having one end fixed to the radio 15' bone. Structure is completed by a wire piece 10". Said structure is assembled by two outer fixation devices 1, one of which provides two blocking elements 3.

As to the reduction of the ulna 15 multiple fracture, it is possible observing the application of outer fixation devices 1 according to inner and outer synthesis modes. Particularly, wire or fiche 10a is substantially introduced along the entire diaphyseal channel, passing through both fracture 16, 17 sections, after that multiple fracture has been reduced. Wires or fiches 10b, 10c and 10d are not implanted along the ulna diaphyseal channel, but rather perpendicular to the same. Said wire or fiche 10a and said wires or fiches 10b, 10c and 10d are bridged by wires 10e and 10f by outer fixation devices 1. the latter provide two blocking elements 3 placed substantially perpendicularly.

In this application, outer fixation devices 1 provide each one two superposed blocking devices 3, permitting blocking two single wires /fiches or two pairs of even diverging wires / fiches.

By this system, it is possible creating stable structures particularly permitting avoiding the sliding of the wires with respect to the bone.

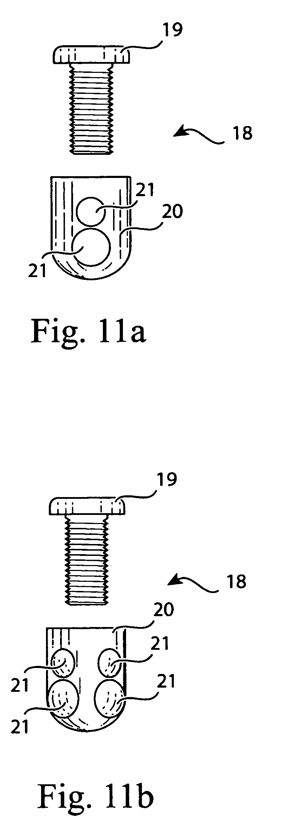

Finally, clamps 18 can be noted in figures 11 a and 11b, providing a screw 19 and a body 20, providing each one two or more possibly parallel or superimposed, open or closed holes 21.

Said clamps 18 are useful when they are used along with outer fixation devices 1, particularly for fixing exceeding parts of wires or fiches 10, for creating more stable assemblies in case a plurality of wires or fiches 10 is employed, for example in case of patella fractures, or to cover wires or fiches 10 ends that could scratch patient and to support insertion of tendons within the bone.

On the basis of the above specification, it can be noted that the basic feature of that present invention is that of providing a fixation devices, that can be used with the Kirschner wire technique, and is quick and efficient, that can be used by known surgery tools, suitable for modern percutaneous and mini-invasive osteosynthesis techniques, that can be used for the arthroscopic treatment of cartilaginous pathology or as a guide for implanting other more bulky synthesis means, such as screws, plates, ecc.

Another advantage of the present invention is that of permitting fixing of two or more diverging wires, avoiding manipulation of the same, and permitting higher precision and higher execution rapidity during surgical intervention.

A further advantage of the present invention is that said fixation device permits a multiplicity of contemporaneous assemblies, along every plane (three-dimensional).

Another advantage of the present invention is that said fixation device can be efficiently used both in the paediatric field and in the veterinary field.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. A multifunctional fixation device (1) for reduction and stabilisation of fractures, adapted to be coupled to two wires (10) insertable into the bone tissue, **characterised in that** said device (1) comprises
a screw element (2) having a threaded stem (8),
one or more blocking element (3), centrally perforated and inserted on said threaded stem (8), having two lateral open seats (5), into which said wires (10) to be fixed are laterally insertable, and two horizontal slits (5"), which extend inwardly from the bottom of each of said seats (5); and
a nut element (4) screwed onto the end of said screw element (2) adapted to tighten said one or more blocking element (3).

2. Fixation device (1) according to claim 1, **characterised in that** said lateral open seats (5) are provided with upper and lower arms (5') enabling said wires (10) to be inserted therein with a snap action.

3. Fixation device (1) according to one of the preceding claims, **characterised in that** said screw element (2) has an enlarged head (6), provided with a socket (7), and said nut element (4) is correspondingly enlarged.

4. Fixation device (1) according to one of the preceding claims, **characterised in that** said two lateral seats (5) are parallel.

5. Fixation device (1) according to one of the preceding claims, **characterised in that** it comprises two superimposed blocking elements (3) having independent orientations.

6. Fixation device (1) according to one of the preceding claims, **characterised in that** said nut element (4) is downwardly closed and capable to receive said threaded stem (8).

7. Fixation device (1) according to one of the preceding claims, **characterised in that** said nut element (4) has a lateral threaded hole (9), suitable to be coupled to a tool.

8. Fixation device (1) according to claim 7, **characterised in that** it comprises a further screw threadedly engageable to said lateral threaded hole (9) for tightening the threading of said threaded stem (8).

9. Fixation device (1) according to one of the claims 7 or 8, **characterised in that** said nut element (4) has provided with a through channel (9'), communicating with said lateral hole (9) extending according to a cord parallel to the diameter of said nut element (4), through which at least a further wire (10) can be introduced.

10. Osteosynthesis assembly, comprising Kirschner wires (10) insertable into the bone tissue according to an inner and/or outer synthesis mode, **characterised in that** it comprises one or more outer fixation devices (1), as defined in claims 1-9, one or more clamps (18) and one or more cap elements (20).

11. Assembly according to claim 10, **characterised in that** said clamps (18) are comprised of a body and of a screw (19), said body, providing one or more through holes (21), within which said wires are introduced and can be fixed by screws (19).

## Patentansprüche

1. Multifunktionelle Fixationsvorrichtung (1) zur Reduktion und Stabilisierung von Frakturen, welche so angepasst ist, dass sie mit zwei Drähten (10), die in das Knochengewebe einführbar sind, gekoppelt ist, **dadurch gekennzeichnet ist, dass** die Vorrichtung (1) umfasst:
ein Schraubenelement (2) mit einem Gewindestamm (8),
mindestens ein Blockierelement (3), das zentral perforiert und an dem Gewindestamm (8) eingeführt ist weiches zwei laterale offene Auflager (5) aufweist, in welche die Drähte (10) zur Befestigung lateral einführbar sind, sowie zwei horizontale Schlitze (5"), welche sich vom Boden von jedem der Auflager (5) nach innen erstrecken, und
ein Gewindemutterelement (4), welches auf das Ende des Schraubenelements (2) aufgeschraubt und so angepasst ist, dass es das mindestens eine Blockierelement (3) festzieht.

2. Fixationsvorrichtung (1) nach Anspruch 1, welche **dadurch gekennzeichnet ist, dass** die lateralen offenen Auflager (5) mit oberen und unteren Armen (5') bereitgestellt sind, welche das Einführen der Drähte (10) darin über einen Einrastvorgang ermöglichen.

3. Fixationsvorrichtung (1) nach einem der vorhergehenden Ansprüche, welche **dadurch gekennzeichnet ist, dass** das Schraubenelement (2) einen vergrößerten Kopf (6) aufweist, der mit einer Fassung (7) bereitgestellt ist, und dass das Gewindemutterelement (4) entsprechend vergrößert ist.

4. Fixationsvorrichtung (1) nach einem der vorhergehenden Ansprüche, welche **dadurch gekennzeichnet ist, dass** die beiden lateralen Auflager (5) parallel angeordnet sind.

5. Fixationsvorrichtung (1) nach einem der vorhergehenden Ansprüche, welche **dadurch gekennzeichnet ist, dass** sie zwei übereinander liegende Blockierelemente (3) umfasst, die unabhängige Orientierungen aufweisen.

6. Fixationsvorrichtung (1) nach einem der vorhergehenden Ansprüche, welche **dadurch gekennzeichnet ist, dass** das Gewindemutterelement (4) nach unten geschlossen und dazu in der Lage ist, den Gewindestamm (8) aufzunehmen.

7. Fixationsvorrichtung (1) nach einem der vorhergehenden Ansprüche, welche **dadurch gekennzeichnet ist, dass** das Gewindemutterelement (4) ein laterales Gewindeloch (9) aufweist, welches dazu geeignet ist mit einem Werkzeug gekoppelt zu werden.

8. Fixationsvorrichtung (1) nach Anspruch 7, welche **dadurch gekennzeichnet ist, dass** sie eine weitere Schraube umfasst, welche über ein Gewinde in dem lateralen Gewindeloch (9) befestigbar ist, um das Gewinde des Gewindestamms (8) festzuhalten.

9. Fixationsvorrichtung (1) nach einem der Ansprüche 7 oder 8, welche **dadurch gekennzeichnet ist, dass** das Gewindemutterelement (4) mit einem Durchgangskanal (9') ausgestattet ist, welcher mit dem lateralen Loch (9) in Verbindung steht, welches sich entsprechend einem Band, das parallel zu dem Durchmesser des Gewindemutterelements (4) verläuft, erstreckt, durch welches mindestens ein weiterer Draht (10) eingeführt werden kann.

10. Osteosyntheseanordnung, welche Kirschner-Drähte (10) umfasst, welche in das Knochengewebe entsprechend einem inneren und/oder äußeren Synthesemodus einführbar sind, **dadurch gekennzeichnet ist, dass** sie mindestens eine äußere Fixationsvorrichtungen (1), wie in Ansprüchen 1-9 definiert, mindestens ein Befestigungselement (18) und mindestens ein Abdeckelement (20) umfasst.

11. Anordnung nach Anspruch 10, **dadurch gekennzeichnet, dass** die Befestigungselemente (18) einen Körper und eine Schraube (19) umfassen, wobei der Körper ein Durchgangsloch oder mehrere Durchgangslöcher (21) bereitstellt, in denen die Drähte eingeführt und durch die Schrauben (19) fixiert werden können.

## Revendications

1. Dispositif de fixation multifonctionnel (1) pour la réduction et la stabilisation des fractures, adapté à l'accouplement à deux broches (10) insérables dans le tissu osseux, **caractérisé en ce que** ledit dispositif (1) comprend
un élément de type vis (2) à tige filetée (8),
un ou plusieurs éléments de blocage (3), perforés en partie centrale et insérés sur ladite tige filetée (8), comportant deux logements ouverts latéraux (5), dans lesquels lesdites broches (10) à fixer sont insérables latéralement, et deux fentes horizontales (5"), qui se prolongent vers l'intérieur depuis le fond de chacun desdits logements (5) ; et
un élément de type écrou (4) vissé à l'extrémité dudit élément de type vis (2) adapté à serrer ledit un ou lesdits plusieurs éléments de blocage (3).

2. Dispositif de fixation (1) selon la revendication 1, **caractérisé en ce que** lesdits logements ouverts latéraux (5) sont pourvus de bras supérieurs et inférieurs (5') permettant auxdites broches (10) d'y être insérées avec une fixation immédiate.

3. Dispositif de fixation (1) selon l'une des revendications précédentes, **caractérisé en ce que** ledit élément de type vis (2) a une tête élargie (6), pourvue d'une cavité (7), et ledit élément de type écrou (4) est élargi proportionnellement.

4. Dispositif de fixation (1) selon l'une des revendications précédentes, **caractérisé en ce que** lesdits deux logements latéraux (5) sont parallèles.

5. Dispositif de fixation (1) selon l'une des revendications précédentes, **caractérisé en ce qu'**il comprend deux éléments de blocage (3) superposés présentant des orientations indépendantes.

6. Dispositif de fixation (1) selon l'une des revendications précédentes, **caractérisé en ce que** ledit élément de type écrou (4) est fermé en bas et apte à recevoir ladite tige filetée (8).

7. Dispositif de fixation (1) selon l'une des revendications précédentes, **caractérisé en ce que** ledit élément de type écrou (4) comporte un trou fileté latéral (9), approprié à l'accouplement à un outil.

8. Dispositif de fixation (1) selon la revendication 7, **caractérisé en ce qu'**il comprend une autre vis dont le filetage permet l'engagement dans ledit trou fileté latéral (9) afin de serrer le filetage de ladite tige filetée (8).

9. Dispositif de fixation (1) selon l'une des revendications 7 ou 8, **caractérisé en ce que** ledit élément de type écrou (4) a été pourvu d'un canal de passage (9'), communiquant avec ledit trou latéral (9) se prolongeant selon un cordon parallèle au diamètre dudit élément de type écrou (4), à travers lequel au moins une autre broche (10) peut être introduite.

10. Ensemble d'ostéosynthèse, comprenant des broches de Kirschner (10) pouvant être insérées dans le tissu osseux selon un mode de synthèse interne et/ou externe, **caractérisé en ce qu'**il comprend un ou plusieurs dispositifs de fixation externes (1), tels que définis dans les revendications 1-9, un ou plusieurs systèmes d'attache (18) et un ou plusieurs éléments à type de coiffe (20).

11. Ensemble selon la revendication 10, **caractérisé en ce que** lesdits systèmes d'attache (18) sont composés d'un corps et d'une vis (19), ledit corps, fournissant un ou plusieurs trous traversants (21), à l'intérieur desquels lesdites broches sont introduites et peuvent être fixées par des vis (19).
